# EUROPEAN PATENT APPLICATION

(11) **EP 3 991 772 A1**
(43) Date of publication of application: **04.05.2022**
(21) Application number: 21205333.4
(22) Date of filing: 28.10.2021
(51) Int. Cl.: A61M 16/00, A61B 5/08, A61B 5/318

(54) **CONTROLLING VENTILATION OF A PATIENT BASED ON FILTERED ELECTROCARDIOGRAM MEASUREMENTS**

(30) Priority: 29.10.2020 US 202063107009 P; 03.05.2021 US 202117306425
(71) Applicant: Biosense Webster (Israel) Ltd, Yokneam, 2066717 (IL)
(72) Inventor: GOVARI, Assaf, 2066717 Yokneam (IL); ALTMANN, Andres Claudio, 2066717 Yokneam (IL); ALGAWI, Yehuda, 2066717 Yokneam (IL); AHARON, Eran, 2066717 Yokneam (IL)
(74) Representative: Carpmaels & Ransford LLP

(57) **Abstract**

A system, method and software product for detecting and controlling respiratory status of a patient based on estimated respiratory status that includes ventilating a patient using a first electrode, configured to be coupled to a chest of the patient at a first position, and to produce a first electrocardiogram (ECG) signal; a second electrode, configured to be coupled to the chest at a second position different from the first position, and to produce a second ECG signal; and a processor, which is configured to: (i) produce a first filtered signal by applying a first filter to the first ECG signal, and a second filtered signal by applying a second filter to the second ECG signal, (ii) estimate, by comparing between the first and second filtered signals, an electrical impedance between the first and second electrodes, which is indicative of a respiratory status of the patient, and (iii) control the ventilation system to apply a ventilation scheme responsively to the estimated electrical impedance.

## Description

### CROSS-REFERENCE TO RELATED APPLICATIONS

This application is related to and claims priority to U.S. Provisional Patent Application 63/107,009, filed on October 29, 2020, whose disclosure is incorporated herein by reference.

### FIELD OF THE INVENTION

The present invention relates generally to patient ventilation methods and systems, and particularly to methods and systems for estimating respiratory status of a patient by comparing between filtered electrocardiogram signals acquired from patient.

### BACKGROUND OF THE INVENTION

Various techniques for estimating respiratory status of a patient have been published in the patent literature.

For example, U.S. Patent 8,790,270 describes a method of monitoring lung ventilation of a subject. The method comprises recording signals from a plurality of sensing location on the chest of the subject, at least a portion of the signals being indicative of a local motion of the chest at a respective sensing location. The method further comprises operating a data processing system to analyze the signals such as to determine a status of the ventilation, thereby to monitor the lung ventilation of the subject.

U.S. Patent 10,092,721 describes a ventilator system that addresses respiratory distress due to the onset of an epidemic or pandemic disease state. In particular, a ventilator system that can be manufactured quickly with minimal skill requirements and employed rapidly in response to epidemic respiratory disease conditions.

U.S. Patent 10,070,804 describes an apparatus for the collection of samples of exhaled air during normal respiration. The apparatus comprising a flow generator, an orally insertable exhalation air receiver, a device for isolating the nasal airways, a sensor for detecting a change in a parameter representing the change from inhalation to exhalation and to transmit said change as a signal, and a control unit adapted to receive said signal and to control said device for isolating the nasal airways.

### SUMMARY OF THE INVENTION

An embodiment of the present invention that is described herein provides a system for estimating a respiratory status of a patient, so as to adjust a ventilation treatment responsively to the clinical condition of the patient, and particularly to changes in the respiratory ability of the patient. For example, the ventilation system is configured to operate in multiple ventilation rates, such as but not limited to (i) a normal-ventilation mode (NVM) and/or a natural respiration of about 15 ventilation cycles per minute, or (ii) a hype-ventilation mode (HVM) of about 100 or more ventilation cycles per minute. In the context of the present disclosure, the term "ventilation rate" refers to a number of ventilation cycles per minute (or any other given period of time) applied to the patient by the ventilation system.

In some embodiments, such ventilation systems, which are configured to estimate or even predict the respiratory condition of the patient, may be used for treating a patient during a medical procedure. For example, during an electrophysiology (EP) procedure performed in a patient heart, or during a thoracic surgery, where the chest has to be stationary. In other cases, such systems may be used for ventilating a patient suffering from an infectious disease affecting the patient respiratory system, such as but not limited to severe acute respiratory syndrome coronavirus (SARS-Cov), also denoted coronavirus disease (COVID-19) .

In some embodiments, a system for estimating the respiratory status of the patient comprises first and second electrodes and a processor connected to a ventilation system, which is ventilating the patient. The first and second electrodes are configured to be coupled to the skin of the patient chest (e.g., using patches) at first and second respective positions, and to produce first and second electrocardiogram (ECG) signals, respectively.

In some embodiments, the processor is configured to: (i) produce a first filtered signal by applying a first filter to the first ECG signal, and a second filtered signal by applying a second filter to the second ECG signal, (ii) estimate, by comparing between the first and second filtered signals, a parameter indicative of an electrical impedance between the first and second electrodes, which is indicative of a respiratory status of the patient, and (iii) control the ventilation system to apply a ventilation scheme or mode responsively to the estimated electrical impedance.

In some embodiments, the first and second filters applied, respectively, to the first and second ECG signals, may comprise any suitable type of low-pass filters (LPFs). For example, the LPF is configured to cut-off the frequencies indicative of the heart rate of the patient, and to pass the frequencies indicative of the respiratory cycle. Based on a regular respiratory rate and heart rate of about 15 per minute and 60 per minute, respectively, the LPF is configured to pass frequencies lower than about 0.8 Hz (corresponding to a respiratory rate lower than about 48 respirations per minute) or even frequencies lower than about 1 Hz when treating a patient having a heart rate higher than about 70 beats per minute (bpm). Note that for patients having a heart rate lower than about 60 bpm, the LPF may be set to cut-off frequencies higher than about 0.6 Hz or 0.7 Hz. The first and second LPFs may differ from one another (e.g., in hardware and/or frequency cut-off setting) or may be similar, depending on the first and second ECG signals.

In some cases, the estimated electrical impedance may alter during the ventilation process, for example, when the patient goes from unconsciousness or anesthesia, to a state of consciousness. In some embodiments, the processor is configured to adjust the number of ventilation cycles per minute and other ventilation parameters responsively to the estimated electrical impedance. For example, when the initial estimated electrical impedance is indicative of unconsciousness or anesthesia and a later estimated electrical impedance indicates or predicts that the patient is regaining consciousness, the processor may adjust one or more ventilation parameters, such as the ventilation rate and flow rate of an oxygen-enriched humidified air (OHA) supplied by the ventilation system.

In other embodiments, the processor is configured to measure, between the first and second electrodes, an electrical impedance not related to the ECG signals. For example, the processor is configured to: (i) control a power supply to apply a voltage gradient between the first and second electrodes, and (ii) measure the electrical impedance between the first and second electrodes.

The inventors found that, in both methods of monitoring described above, altering impedance is indicative of muscle contraction in patient chest and/or in patient diaphragm, which may indicate or even predict a change in the patient's state of consciousness.

In some embodiments, the ventilation system comprises (i) an OHA tube, which is configured to flow OHA produced by a compressor and humidifier toward lungs of the patient, (ii) an exhaust tube, which is configured to flow exhaust air exhaled from the lungs to an air evacuation system (AES) of a hospital, and (iii) an intubation tube, which is configured to flow the OHA and the exhaust air between the lungs and the OHA and exhaust tubes. The intubation tube comprising a distal end, configured to be inserted into a trachea of the patient, and a proximal end, configured to be connected to the OHA and exhaust tubes. In the context of the present disclosure and in the claims, the term "exhaust air" refers to air flowing from the lungs into the air evacuation system of the hospital. Note that the term "exhaled" is applicable when the patient can exhale the air from the lungs, otherwise, the term exhaled may be replaced with a term "sucked" referring to suctioning, typically part of the air, from the patient lungs.

In some embodiments, the ventilation system may comprise additional devices for controlling parameters of the OHA flowing into patient lungs and exhaust air exhaled from the patient lungs. Such devices may comprise sensors, valves and flow regulators controlled by the processor.

In some embodiments, in response to altered estimated electrical impedance, the processor is configured to switch between the NVM and HVM modes by controlling the frequency at which the compressor and humidifier are producing the OHA. For example, when the estimated electrical impedance exceeds a predefined threshold, the processor may switch between the ventilation modes.

In some embodiments, the present invention is also directed to a computer software product, comprising a non-transitory computer-readable medium in which program instructions are stored, which instructions, when read by a processor for a ventilator system for use with at least a first and second electrodes coupled externally to the chest of a patient, the processor programmed to perform the steps of: (i) producing first and second filtered signals received from the at least the first and second electrodes; and (ii) estimate, by comparing between the first and second filtered signals, an indication of the electrical impedance between the at least first and second electrodes, and (iii) control the ventilation system to apply a ventilation scheme or ventilation mode responsively to the estimated electrical impedance. In some embodiments according the present invention the computer software product further comprises (i) a normal-ventilation mode (NVM) at a respiration of about 15 ventilation cycles per minute and (ii) a hype-ventilation mode (HVM) of about 100 or more ventilation cycles per minute.

The disclose techniques improve the responsiveness and even the prediction of a ventilation system to a change in the patient's respiratory condition, and therefore, may save the patient's life and/or improve the quality of medical procedures performed on the patient's respiratory system and/or the patient's heart.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a ventilation system having a subsystem for detecting respiratory status of a patient, in accordance with an embodiment of the present invention; and
Fig. 2 is a flow chart that schematically illustrates a method for controlling ventilation based on estimated respiratory status of patient, in accordance with an embodiment of the present invention.

### DETAILED DESCRIPTION OF EMBODIMENTS

### OVERVIEW

Ventilation systems are used for mechanically assisting or replacing autonomic breathing when a patient cannot breathe by himself/herself adequately. Such systems may be operated in different ventilation modes in accordance with the clinical situation of the patient as will be described below. In practice, when a patient attempts to breath, it is important not to resist such breathing attempts. For example, when the ventilation system supplies air and the patient coughs, the ventilator should be controlled to hold the air supply, and resume after the patient stops coughing and/or needs ventilation. Therefore, it is important to identify or even predict when the patient is trying, or will soon be trying, to breathe independently.

### SYSTEM DESCRIPTION

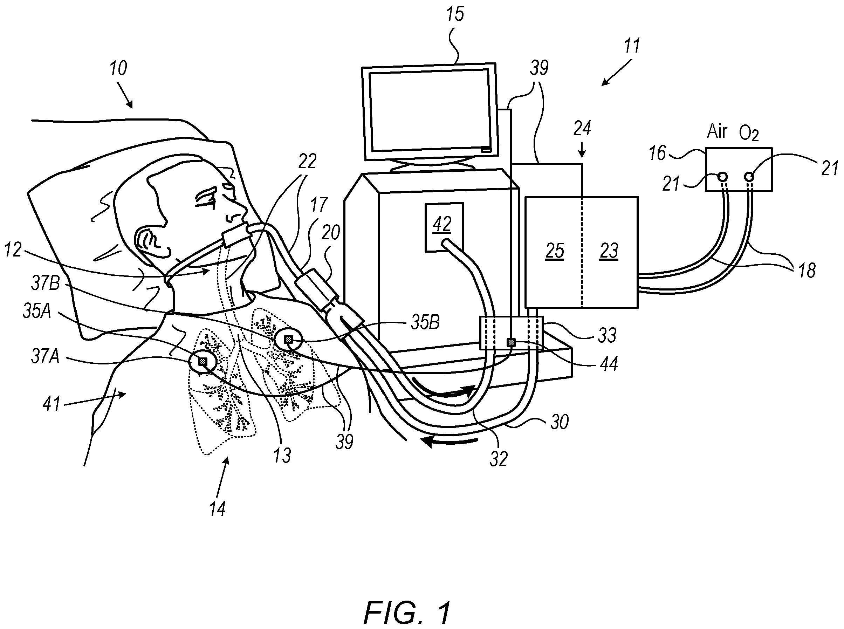

Fig. 1 is a schematic, pictorial illustration of a ventilation system 11, in accordance with an embodiment of the present invention.

In some embodiments, ventilation system 11 comprises an oxygen-enriched humidified air (OHA) supply subsystem (OHAS) 24 comprising an air compressor 23 and a humidifier 25. Air compressor 23 is configured to compress a preassigned gas mixture to a preassigned pressure described below.

In some embodiments, ventilation system 11 comprises tubes 18 connected between OHAS 24 and a hospital supply 16. In the present example, air compressor 23 is configured to receive, via outlets 21 of hospital supply 16, oxygen (O₂) and air so as to produce the aforementioned OHA at a preassigned mixture (e.g., defined in volumetric percentage) and a preassigned pressure. In the present example, the produced OHA has a typical percentage of oxygen between about 21% and 100% and a preassigned pressure between about zero CmH₂O (when a patient 10 is self-breathing) and 50 CmH₂O.

In some embodiments, humidifier 25 is configured to harvest moisture from the environment or to receive water from any suitable source, so as to set a preassigned humidity of the OHA. For example, humidifier 25 is configured to supply OHA at a selected humidity level between about 40% and 100%, or at any other suitable humidity. In the present example, OHAS 24 comprises reusable air compressor 23 and humidifier 25, which may have to undergo a minimal or no cleaning or sterilization between ventilations of different patients 10.

In some embodiments, system 11 comprises an intubation tube 22 having a distal end 13, which is configured to be inserted into a trachea 12 of patient 10, and a proximal end 17, which is connected to a bifurcation adaptor 20.

In some embodiments, ventilation system 11 comprises an OHA tube 30, which is coupled between bifurcation adaptor 20 and OHAS 24, and is configured to flow the OHA produced by air compressor 23 and humidifier 25 or OHAS 24, via intubation tube 22, toward lungs 14 of patient 10. Note that lungs 14 are exposed by depiction in Fig. 1 and shown for the sake of more clear presentation, noting that lungs 14 in the thoracic cavity of the patient 10 which is covered with natural tissue (e.g., pleura, bone, muscles, skin, etc.) removed from Fig. 1 for the sake of conceptual clarity.

In some embodiments, system 11 further comprises an exhaust tube 32, which is coupled between bifurcation adaptor 20 and an air evacuation system (AES) 42 of the hospital. Exhaust tube 32 is configured to flow exhaust air exhaled from lungs 14, via intubation tube 22, toward AES 42.

Note that the term "exhaled" is applicable when patient 10 can independently exhale at least a portion of the air from lungs 14. Otherwise, the term exhaled may be replaced with the terms "expiration", "evacuated", "aspirated" or "sucked" referring to "expiration", evacuation, aspiration or suctioning, typically part of the air, from lungs 14 of patient 10.

In some embodiments, ventilation system 11 comprises multiple patches, in the present example, two patches 37A and 37B coupled externally, e.g., to the skin, of a chest 41 of patient 10. Each patch comprises one or more electrodes, in the present example, patch 37A comprises an electrode 35A and patch 37B comprises an electrode 35B.

In some embodiments, electrode 35A is configured to produce a first electrocardiogram (ECG) signal, and electrode 35B is configured to produce a second ECG signal.

In some embodiments, ventilation system 11 comprises a control unit 33, which is configured to monitor and control parameters of the ventilation process, such as but not limited to the ventilation rate, gas mixture (e.g., oxygen), flow rate (e.g., between about 10 liter per minute (LPM) and 60 LPM, or at any other suitable flow rate) and the humidity of the OHA flowing into lungs 14. In the present example, control unit 33 comprises a processor 44, which is electrically connected via electrical leads 39 (or suitable cables), *inter-alia,* to OHAS 24, a display 15, electrodes 35A and 35B and additional devices (not shown) such as sensors and valves for controlling ventilation system 11.

In some embodiments, processor 44 is configured to control air compressor 23 and humidifier 25 of OHAS 24, by setting the preassigned thresholds, such as gas mixture (having oxygen percentage between about 21% and 100%), pressure (between about 0 CmH₂O and 100 CmH₂O) and humidity (e.g., between about 40% and 100%) of the OHA, and by controlling the frequency of the ventilation rate carried out by compressor 23.

In some embodiments, system 11 comprises one or more displays, such as but not limited to display 15 or a display (not shown) of control unit 33.

In some embodiments, processor 44 is configured to display, e.g., on display 15, one or more parameters indicative of the ventilation rate, flow rate, and/or humidity and/or any other suitable parameter of the OHA as well as the exhaust air flowing in tubes 30 and 32 as described above.

In some embodiments, based on the first and second ECG signals received from electrodes 35A and 35B, respectively, processor 44 is configured to produce a first filtered signal by applying a first low-pass filter (LPF) to the first ECG signal, and a second filtered signal by applying a second LPF to the second ECG signal.

In some embodiments, the LPF is configured to cut-off the frequencies indicative of the heart rate of the patient, and to pass the frequencies indicative of the respiratory cycle. Based on a regular respiratory rate and heart rate of about 15 per minute and 60 per minute, respectively, the LPF is configured to pass frequencies lower than about 0.8 Hz (corresponding to a respiratory rate lower than about 48 respirations per minute) or even frequencies lower than about 1 Hz when treating a patient having a heart rate higher than about 70 beats per minute (bpm). Note that for patients having a heart rate lower than about 60 bpm, the LPF may be set to cut-off frequencies higher than about 0.6 Hz or 0.7 Hz. The first and second LPFs may differ from one another (e.g., in hardware and/or frequency cut-off setting) or may be similar, depending on the first and second ECG signals.

In some embodiments, processor 44 is configured to estimate, by comparing between the first and second filtered signals, an electrical impedance between the first and second electrodes. Note that the estimated electrical impedance is indicative of the respiratory status of patient 10.

In some embodiments, processor 44 is configured to control ventilation system 11 to apply a ventilation scheme or mode responsively to the estimated electrical impedance.

In other embodiments, processor 44 is configured to apply any other suitable type of filters to the ECG signals, in addition to or instead of the aforementioned first and second LPFs.

In some cases, the estimated electrical impedance may alter during the ventilation process, for example, when the patient goes from an unconscious or anesthesia to a conscious state. In some embodiments, processor 44 is configured to adjust the ventilation rate (i.e., the number of ventilation cycles per minute) and other ventilation parameters responsively to the estimated electrical impedance. For example, when the initial estimated electrical impedance is indicative of unconsciousness or anesthesia and a later estimated electrical impedance indicates or predicts that patient 10 is regaining consciousness, processor 44 may adjust one or more ventilation parameters, such as the ventilation rate of the OHA supplied by compressor 23 of OHAS 24.

In such embodiments, processor 44 is configured to switch between a hyper-ventilation mode (HVM) and a normal-ventilation mode (NVM) of ventilation system 11. In other words, based on the estimated electrical impedance, processor 44 is configured to detect or even predict that patient 10 is regaining consciousness, and therefore may reduce the ventilation rate from about 100 ventilation cycles per minute to about 15 ventilation cycles per minute.

In the context of the present disclosure and in the claims, the terms "about" or "approximately" for any numerical values or ranges indicate a suitable dimensional tolerance that allows the part or collection of components to function for its intended purpose as described herein.

In some embodiments, processor 44 is configured to switch between the HVM and NVM modes by controlling the frequency at which air compressor 23 and humidifier 25 of OHAS 24 are producing the specified attributes of the OHA produced by OHAS 24.

Typically, processor 44 comprises a general-purpose processor, which is programmed in software to carry out the functions described herein. The software may be downloaded to the processor in electronic form, over a network, for example, or it may, alternatively or additionally, be provided and/or stored on non-transitory tangible media, such as magnetic, optical, or electronic memory.

This particular configuration of ventilation system 11 is shown by way of example, in order to illustrate certain problems that are addressed by embodiments of the present invention and to demonstrate the application of these embodiments in enhancing the performance of such a system. Embodiments of the present invention, however, are by no means limited to this specific sort of example system, and the principles described herein may similarly be applied to other sorts of ventilation systems and/or to any other sorts of ventilation subsystems used, for example, as modules of a system for conducting any medical procedure.

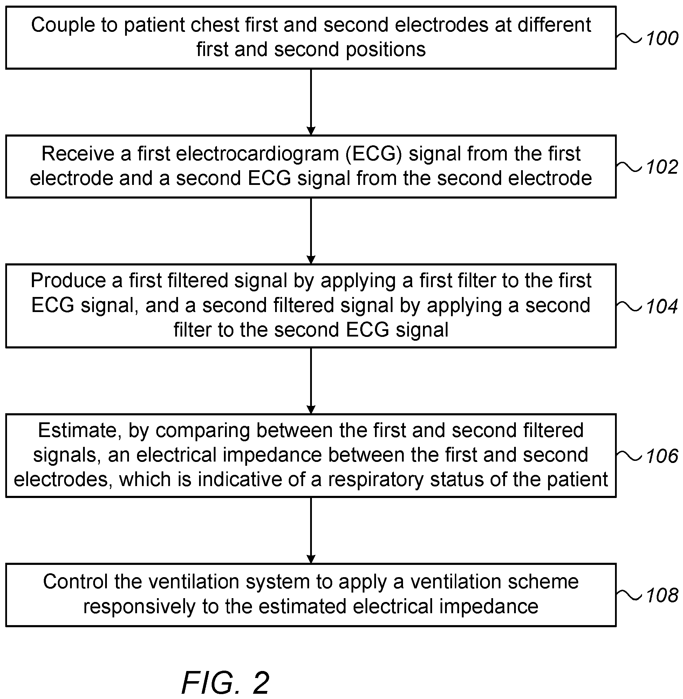

In some embodiments, a processor of the ventilation system may have a software, which is configured to carry out the operations described above, and in the method described in Fig. 2 below. In particular, the processor of the ventilation system is configured to: (i) produce the first and second filtered signals, e.g., using the LPFs, (ii) estimate, by comparing between the first and second filtered signals, an indication of the electrical impedance between the first and second electrodes, and (iii) control the ventilation system to apply a ventilation scheme or ventilation mode responsively to the estimated electrical impedance.

### CONTROLLING VENTILATION BASED ON ESTIMATED RESPIRATORY

### STATUS OF PATIENT

Fig. 2 is a flow chart that schematically illustrates a method for controlling a ventilation rate, based on estimated respiratory status of patient 10, in accordance with an embodiment of the present invention. The method begins at a patch coupling step 100, with coupling to different positions on the skin of chest 41, patches 37A and 37B having electrodes 35A and 35B, respectively, as described in Fig. 1 above.

At an ECG-signal receiving step 102, processor 44 receives the first ECG signal from electrode 35A and the second ECG signal from electrode 35B, as described in Fig. 1 above.

At a filtered-signal producing step 104, processor 44 is configured to produce the first filtered signal by applying a first LPF to the first ECG signal, and a second filtered signal by applying a second LPF to the second ECG signal. Note that processor 44 may apply any suitable filter to the ECG signals, in addition to or instead of the LPFs, and the first and second LPFs may be similar or may differ from one another, depending on the attributes of the first and second ECG signals.

At an electrical impedance estimation step 106, processor 44 is configured to estimate, by comparing between the first and second filtered signals, the electrical impedance between electrodes 35A and 35B, which is indicative of the respiratory status of patient 10.

At a ventilation control step 108 that concludes the method, processor 44 is configured to control ventilation system 11 to apply a ventilation scheme or mode responsively to the estimated electrical impedance, as described in detail in Fig. 1 above.

In some embodiments, processor 44 is configured to adjust the number of ventilation cycles per minute responsively to the estimated electrical impedance. For example, when the estimated electrical impedance is indicative of patient 10 regaining consciousness, processor 44 is configured to reduce the ventilation rate from about 100 ventilation cycles per minute to about 15 ventilation cycles per minute. Similarly, when the estimated electrical impedance is indicative of patient 10 trying to breathe independently, processor 44 is configured to hold the ventilation and let patient 10 breathe naturally using his respiratory system.

Although the embodiments described herein mainly address ventilation system used for treating patients having an infectious disease affecting the patient respiratory system, the methods and systems described herein can also be used in other medical applications. For example, the methods and systems described above may be used in electrophysiology (EP) procedures applied to patient heart, in a ventilation during a thoracic surgery, in which the patient chest has to be immobile, in a procedure associated with ventilating lungs of an infant, and in other suitable medical procedures associated with the patient respiratory system.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. A method, comprising:
coupling, to a chest of a patient at least partially ventilated by a ventilation system for providing oxygen-enriched humidified air (OHA) to patient, wherein the system comprises a first electrode at a first position, and coupling to the chest a second electrode at a second position, different from the first position;
receiving a first electrocardiogram (ECG) signal from the first electrode, and a second ECG signal from the second electrode;
producing (i) a first filtered signal by applying a first filter to the first ECG signal, and (ii) a second filtered signal by applying a second filter to the second ECG signal;
estimating, by comparing between the first and second filtered signals, an electrical impedance between the first and second electrodes, wherein the electrical impedance is indicative of a respiratory status of the patient; and
controlling the ventilation system to apply a ventilation scheme for the OHA responsively to the estimated electrical impedance.

2. The method according to claim 1, wherein applying the first and second filters comprises applying a first low-pass filter (LPF) to the first ECG signal and applying a second LPF to the second ECG signal.

3. The method according to claim 1, wherein controlling the ventilation system comprises adjusting a number of ventilation cycles per minute responsively to the estimated electrical impedance.

4. The method according to claim 1, and comprising measuring, between the first and second electrodes, an additional electrical impedance,

5. The method according to claim 4, and comprising controlling the ventilation system based on the estimated electrical impedance and the measured additional electrical impedance.

6. The method according to claim 1, further comprising setting preassigned thresholds for at least one of gas mixture, pressure and humidity for the OHA of the ventilation system, preferably further comprising a preassigned threshold for gas mixture having oxygen percentage between about 21% and 100%), more preferably further comprising a preassigned threshold for pressure between about 0 CmH2O and 100 CmH2O, still more preferably further comprising a preassigned threshold for humidity of the OHA between about 40% and 100%.

7. The method according to claim 1, further comprising of controlling the frequency of a ventilation rate of the ventilation system, preferably controlling the frequency of the ventilation rate indicative of the respiratory cycle for the patient, more preferably further comprising a ventilation scheme comprising multiple ventilation modes wherein each ventilation mode is a different ventilation rate, still more preferably further comprising (i) a normal-ventilation mode (NVM) at a respiration of about 15 ventilation cycles per minute and (ii) a hype-ventilation mode (HVM) of about 100 or more ventilation cycles per minute, even still more preferably further comprising switching between the hyper-ventilation mode (HVM) and the normal-ventilation mode (NVM) for the ventilation system.

8. A system for ventilating a patient, the system comprising:
a first electrode, configured to be coupled to a chest of the patient at a first position, and to produce a first electrocardiogram (ECG) signal;
a second electrode, configured to be coupled to the chest at a second position different from the first position, and to produce a second ECG signal; and
a processor, which is configured to: (i) produce a first filtered signal by applying a first filter to the first ECG signal, and a second filtered signal by applying a second filter to the second ECG signal, (ii) estimate, by comparing between the first and second filtered signals, an electrical impedance between the first and second electrodes, which is indicative of a respiratory status of the patient, and (iii) control the ventilation system to apply a ventilation scheme for providing oxygen-enriched humidified air (OHA) to the patient responsively to the estimated electrical impedance.

9. The system according to claim 8, wherein the processor is configured to apply: (i) a first low-pass filter (LPF) to the first ECG signal, and (i) a second LPF to the second ECG signal.

10. The system according to claim 8, wherein the processor is configured to adjust a number of ventilation cycles per minute responsively to the estimated electrical impedance.

11. The system according to claim 10, further comprising (i) a normal-ventilation mode (NVM) at a respiration of about 15 ventilation cycles per minute and (ii) a hype-ventilation mode (HVM) of about 100 or more ventilation cycles per minute.

12. The system according to claim 8, wherein the processor is configured to measure, between the first and second electrodes, an additional electrical impedance.

13. The system according to claim 8, wherein the processor is configured to control the ventilation system based on the estimated electrical impedance and the measured additional electrical impedance.

14. A computer software product, comprising a non-transitory computer-readable medium in which program instructions are stored, which instructions, when read by a processor for a ventilator system for use with at least a first and second electrodes coupled externally to the chest of a patient, the processor programmed to perform the steps of:
(i) producing first and second filtered signals received from the at least the first and second electrodes; and
(ii) estimate, by comparing between the first and second filtered signals, an indication of the electrical impedance between the at least first and second electrodes, and (iii) control the ventilation system to apply a ventilation scheme or ventilation mode responsively to the estimated electrical impedance.

15. The computer software product according to claim 14, further comprising (i) a normal-ventilation mode (NVM) at a respiration of about 15 ventilation cycles per minute and (ii) a hype-ventilation mode (HVM) of about 100 or more ventilation cycles per minute.
